# EUROPEAN PATENT APPLICATION

(11) **EP 1 714 649 A2**
(43) Date of publication of application: **25.10.2006**
(21) Application number: 06016819.2
(22) Date of filing: 30.09.1998
(51) Int. Cl.: A61K 31/195, A23L 1/305, A61K 9/08, A61P 1/16

(54) **L-Valine for hepatic diseases**

(30) Priority: 30.09.1997 JP 26579397
(62) Divisional of application: 98945536.5
(71) Applicant: CHUGAI SEIYAKU KABUSHIKI KAISHA, Tokyo, 115-8543 (JP)
(72) Inventor: Satomi, Susumu c/o Tohoku University, Sendai-shi Miyagi 980-8574 (JP); Doi, Hideyuki c/o Tohoku University, Sendai-shi Miyagi 980-8574 (JP); Chin, Masahiro c/o Tohoku University, Sendai-shi Miyagi 980-8574 (JP); Komatsu, Hiromichi, Gotenba-shi Shizuoka 412-8513 (JP); Koga, Hiroshi, Chuo-ku Tokyo 104-8301 (JP)
(74) Representative: Vossius & Partner

(57) **Abstract**

Compositions that contain valine as an active ingredient but which are entirely free of other amino acids or substantially free of amino other acids as an active ingredient are used as drugs or foods for treating or ameliorating hepatic diseases, whereupon less side effects are caused than in the conventional regimens of pharmacotherapy and yet the compositions ameliorate, palliate or gain recovery from symptoms and abnormalities that are caused by such hepatic diseases, for example, fever, lassitude, loss of appetite, vomiting stomachache, ascites and pleural effusion, or complications of hepatic disease (not including hepatic encephalopathy).

## Description

### TECHNICAL FIELD

This invention relates to compositions for treating hepatic diseases or improving the hepatic function that are characterized by containing valine as an active ingredient and being substantially free of other amino acids as an active ingredient. More specifically, the invention relates to pharmaceutical or food compositions that contain valine as an active ingredient capable of treating or ameliorating hepatic diseases such as acute hepatitis, hepatic insufficiency, chronic hepatitis and cirrhosis but which are substantially free of other amino acids as an active ingredient.

### BACKGROUND ART

Various amino acid preparations are conventionally used against hepatic diseases such as hepatic insufficiency and cirrhosis. For example, amino acid preparations such as Aminoleban (registered trademark), Morihepamin (registered trademark), Aminoleban (registered trademark) EN, Hepan (registered trademark) ED and Livact (registered trademark) granule are used for such purposes as ameliorating hepatic encephalopathy and hypoalbuminemia that accompany hepatic diseases such as cirrhosis and hepatic insufficiency. In fact, however, these amino acid preparations are used not for direct treatment or amelioration of the mentioned hepatic diseases but rather in anticipation of an improvement in impaired nutrition due to hepatic diseases, namely, for such purposes as improving nitrogen metabolism by correcting the imbalance in plasma amino acids and lowering the blood ammonia level. In addition, these preparations are mixtures of amino acids and single amino acids are little known to be capable of ameliorating the mentioned hepatic diseases. Referring to the official gazette of Examined Japanese Patent Publication No. 29446/1982, it is taught that an injection of L-valine, when administered alone, is useful in the treatment of hepatic encephalopathy; however, hepatic encephalopathy is one of the complications of worsened hepatic disease and toxic substances such as ammonia that accumulate in blood impair the central nervous system to cause various neurotic symptoms; hence, hepatic encephalopathy is different from "hepatic disease" in the sense of term used in the present invention. What is more, the official gazette, supra, makes no suggestion that L-valine is capable of direct treatment or amelioration of hepatic diseases per se. As a matter of fact, hepatic encephalopathy is currently treated with blood ammonia lowering agents such as lactulose and there have been reported no cases of using therapeutics for hepatic diseases in the treatment of hepatic encephalopathy, of which fact shows that the present invention is by no means easy to derive from the official gazette, supra.

The present inventors previously found that valine had a capability of regenerating hepatocytes in the liver that remained after hepatectomy on patients with hepatic diseases such as hepatopathy and cirrhosis and later filed a patent application (see the official gazette of Unexamined Japanese Patent Publication No. 67628/1996). The official gazette, supra, has a disclosure to the effect that valine is effective in regenerating hepatocytes but it neither teaches nor suggests that valine is effective against hepatic diseases per se such as hepatitis and cirrhosis.

Several drugs are known as therapeutics for hepatic diseases such as chronic hepatitis and cirrhosis but none of them are completely satisfactory in terms of effectiveness and safety. Take, for example, glycyrrhizin preparations such as Minophagen C which are currently used against the mentioned hepatic diseases; however, since these preparations are inactivated in the intestines, they are primarily used as injections and do not hold much promise for efficacy if administered perorally. Side effects of the glycyrrhizin preparations due to their aldosterone-like action have also been reported and they include increased blood pressure, hypokalemia and the tendency toward water retention due to sodium retention; these side effects are particularly problematic in severe cases of hepatopathy involving ascites and the like or when prolonged administration is done.

According to the official gazette of Examined Japanese Patent Publication No. 29446/1982, L-valine is an amino acid which, when applied singly, is useful in the treatment of hepatic encephalopathy. However, this utility is based on the lowering of the ammonia concentration due to the degradation of valine to succinyl coenzyme A which is a participating member of the citric acid cycle and it is only applicable to hepatic encephthalopathy characterized by a marked increase in the ammonia concentration of tissue. As already mentioned, hepatic encephalopathy is not a disease in the liver itself but is one of the complications of worsened hepatic disease; hence, the official gazette, supra, does not teach direct treatment or amelioration of hepatic diseases per se.

### DISCLOSURE OF INVENTION

Under these circumstances, the present inventors have been conducting intensive studies with a view to-finding a therapeutic for hepatic diseases that is highly effective, that has no safety problems and that is effective not only in injection but also in oral administration. It has recently been found that when valine, a kind of amino acids, is administered perorally or parenterally in the entire or substantial absence of other amino acids as an active ingredient, a satisfactory ameliorating action is exhibited for hepatic diseases such as hepatic insufficiency, acute hepatitis, chronic hepatitis and cirrhosis without any problems in terms of safety. This finding has led to the accomplishment of the present invention.

As will be demonstrated in the working examples given later in this specification, the efficacy of valine against hepatic diseases can be verified in animal models of hepatic disease or human patients with hepatic disease. For acute hepatitis and hepatic insufficiency, evaluation was made with models of drug (e.g. galactoxamine and carbon tetrachloride) induced acute hepatopathy and 90% hepatectomized models of hepatic insufficiency; for chronic hepatitis and cirrhosis, evaluation was made with models of drug (e.g. carbon tetrachloride) induced chronic hepatopathy. In these models, valine was found to be effective. It was also demonstrated that when administered perorally to patients with chronic hepatitis, valine improved the hepatic function (e.g. GOT GPT and platelet count). These facts demonstrated that valine is useful against hepatic diseases in human and other animals.

### BEST MODE FOR CARRYING OUT THE INVENTION

Valine to be used in the present invention may be a commercial product, or it may be synthesized by one or other method. Valine may be used in any one of D-, L- and DL-forms, with the L-form being particularly preferred.

In the present invention, valine is expected to be capable of treating or ameliorating various hepatic diseases including acute hepatitis, chronic hepatitis, hepatic insufficiency and cirrhosis and particularly good therapeutic efficacy is exhibited in acute hepatitis, hepatic insufficiency and the like. Types of hepatitis for which valine's therapeutic efficacy is expected include acute and chronic cases of hepatitis that are caused by hepatitis A, B, C, D, E, and other viruses. Cases of hepatic insufficiency include acute hepatic insufficiency and chronic hepatic insufficiency. The valine of the present invention is also effective in ameliorating, palliating or gaining recovery from various symptoms and abnormalities that are associated with the above-mentioned diseases including hepatitis and hepatic insufficiency, or complications that are not influenced by ammonia concentration, for example, fever, lassitude, loss of appetite, vomiting, stomachache, ascites and pleural effusion. The complications contemplated here do not include hepatic encephalopathy which is influenced by ammonia concentration.

The composition of the present invention for treating or ameliorating hepatitic diseases may be administered either perorally or parenterally by such routes as intrarectal, subcutaneous, intrathecal, intramuscular, intravenous, intra-arterial and transcutaneous routes. Peroral or intravenous administration is preferred.

For in vivo administration, valine according to the present invention is preferably formulated in an appropriate dosage form and exemplary preparations that can be used include tablets, powders, granules, subtilized granules, pills, capsules, lozenges, chewable preparations, liquid preparations, emulsions, suspensions, suppositories, syrups, lotions, ointments and cataplasms. Pharmaceutical formulating procedures to give these dosage forms may be carried out using pharmaceutically acceptable carriers, vehicles and other appropriate additives.

A liquid preparation is a dosage form that is preferred for intravenous administration of the composition of the present invention for treating or ameliorating hepatic diseases. To make liquid preparations, solvents may be used as exemplified by purified water, physiological saline, alcohols (e.g. ethanol, propylene glycol, glycerol, and polyethylene glycol) and triacetin. The above-mentioned preparations may be supplemented with auxiliary agents such as antiseptics, moistening agents, emulsifers, dispersants and stabilizers. The composition of the present invention may also be administered as a suspension.

Solid preparations such as tablets, pills, powders, granules, subtilized granules, lozenges and chewable preparations can be made by conventional methods using carriers such as sodium hydrogencarbonate, calcium carbonate, starch, sucrose, mannitol and carboxy-methylcellulose, and other additives such as calcium stearate, magnesium stearate and glycerol. Enteric preparations may also be made with an enteric coat being applied by spraying solutions, in either organic solvents or water, of enteric substances such as cellulose acetate phthalate, hydroxypropylmethylcellulose phthalate, polyvinylalcohol phthalate, styrene-maleic anhydride copolymer and methacrylic acid-methyl methacrylate copolymer. Pharmaceutically acceptable carriers typically include optional auxiliary agents, fragrances, stabilizers or antiseptics. The composition of the present invention for treating or ameliorating hepatic diseases may be used in combination with infusion preparations such as total parenteral nutrition; alternatively, valine may be added to other infusion preparations.

The composition of the present invention for treating or ameliorating hepatic diseases may also be applied as food for ameliorating the liver function. If this is the case, valine may be directly added to existing foods, beverages or the like; alternatively, valine may be directly added to confectionery including gum, candies, jelly, "Gummi", cookies, biscuits and chocolate, soft drinks such as juice, dairy products such as cheese, butter and yogurt, processed agriproducts such as ice cream and ham, processed marine products such as chikuwa and hampen (both Japanese), noodles such as soba and udon (both Japanese), processed wheat flour products such as bread and cake, canned food, and seasonings such as salt, pepper, sugar and artificial sweeteners, otherwise, valine may be mixed with the food being processed, the mixture being further processed. The composition of the present invention also finds utility as a so-called "designated health-promoting medicine" for ameliorating the hepatic function. If valine is to be added or mixed with food, it may be used in a solid form such as powder, granules or subtilized granule, alternatively, it may be in a liquid form. If valine is to be processed into food, the procedure may be based on any conventional food processing methods.

The composition of the present invention for treating or ameliorating hepatic diseases contains valine as an active ingredient and unlike heretofore well-known, other amino acid mixed preparations, it is either entirely free of other amino acids or substantially free of other amino acids as an active ingredient (i.e., one to be contained in the therapeutic for hepatic diseases). Thus, the composition of the present invention is outstanding in that it has dissolved the heretofore felt concern over effectiveness and safety.

If the composition of the present invention for treating or ameliorating hepatic diseases is to be used as a medicine, it is used in patients suffering from hepatic diseases such as acute hepatitis, hepatic insufficiency, chronic hepatitis and cirrhosis. Depending on such factors as the sex of the patient, his or her physique, constitution, age, symptoms and the dosage form to be administered, the dose of the' composition can appropriately be selected from the range of 0.1 - 300 g, preferably 1 - 100 g, in terms of the amount of valine as an active ingredient. Depending on such factors as the symptoms of the patient and the dosage form to be administered, the frequency of administration is suitably one to several occasions per day.

### Examples

The following examples are provided for the purpose of further illustrating the present invention but are in no way to be taken as limiting.

### Example 1: Action on Acute Hepatopathy

### 1) Experimental

Crj: Donryu male rats aged 8 - 9 weeks and weighing 250 g or so were used. During the experiment, the rats were given a dedicated feed MF (product of Oriental Yeast) and water ad libitum. D-Galactosamine HCl (product of Sigma) was dissolved in physiological saline, adjusted to pH 7.0 with 1 N NaOH and used as a solution containing 100 mg of D-galactosamine (hereinafter referred to simply as "galactosamine") per milliliter. Carbon tetrachloride (product of Wako Pure Chemical Industries) was used as a 50% (v/v) solution in olive oil (product of Wako Pure Chemical Industries). After 12-h fasting, the rats were administered intraperitoneally with galactosamine in a volume of 10 mL/kg or subcutaneously with the carbon tetrachloride solution at a volume of 4 mL/kg to induce hepatopathy. Immediately after the administration, the rats were anesthetized with ether and a catheter was inserted into the right cervical vein of each animal and retained in the central vein. The catheter in the central vein was passed under the skin to connect the blade bones, fitted with a harness, passed through a protective coil and connected to a swivel (Bio-Cannula of Biomedica). The rats were transferred into a metabolic cage and subjected to the experiment under a non-anesthetized and unconstrained condition. The experimental fluids were administered constantly with the pumping speed set at 100 mL/kg/day. The experimental fluids were administered for 1 - 4 days. The treated groups were two, one being a control group administered with a lactated Ringer's injection [Lactec (registered trademark) of Otuka Seiyaku] and the other being an L-Val group administered with valine (L-valine added to lactated Ringer's injection to a concentration of 16.88 g/L). A non-treated (NT) group was also set. After the end of administration of the experimental fluids, the rats were anesthetized with ether and blood was taken from the abdominal aorta; thereafter, autopsy was done to collect liver samples.

### 2) Results

### a) Rat model of galactosamine-induced acute hepatopathy

The results are shown in Table 1.

The administration of galactosamine caused hepatopathy in rat but it could be ameliorated by administration of L-valine. The Fischer ratio [BCAA (Val, Leu, Ile)/AAA (Phe, Thy, Trp)] also improved.

**Table 1**

| Efficacy in Rat Model of Glactosamine-Induced Acute Hepatopathy (with experimental fluids administered for one day) | | | | | | |
|---|---|---|---|---|---|---|
| | TP (g/dl) | Alb (g/dl) | GOT (IU/l) | GPT (IU/l) | LDH (IU/l) | Fischer ratio BCAA/AAA |
| NT group (n=6) | 5.6±0.1 | 2.7±0.1 | 136.0± 52.8 | 69.5± 19.0 | 813.0± 310.5 | 2.63±0.16 |
| Control Group (n=5) | 4.6±0.2 ** | 2.4±0.1 ** | 1461.8± 721.5 ** | 938.6± 523.2 ** | 2213.0± 1050.7 ** | 2.23±0.26 * |
| L-Val group (n=6) | 4.9±0.3 **,## | 2.5±0.1 | 760.2± 215.4 *,## | 415.7± 180.8 *,## | 973.7± 362.0 ## | 6.42±0.40 **,## |

The values are mean ± SD; * and ** represent statistical significance at levels of p < 0.05 and p < 0.01, respectively, as compared with the NT group, # and ## represent statistical significance at levels of p < 0.05 and p < 0.01, respectively, as compared with the control group; data was obtained by Student's t-test.

### b) Rat model of carbon tetrachloride-induced acute hepatopathy

The administration of carbon tetrachloride caused hepatopathy in rat but could be ameliorated by administration of L-valine. The Fischer ratio [BCAA (Val, Leu, Ile)/AAA (Phe, Thy, Try)] also improved.

**Table 2**

| Efficacy in Rat Model of CCl₄-Induced Acute Hepatopathy (with experimental fluids administered for two days) | | | | | | |
|---|---|---|---|---|---|---|
| | TP (g/dl) | Alb (g/dl) | GOT (IU/l) | GPT (IU/l) | LDH (IU/l) | Fischer ratio BCAA/AAA |
| NT group (n=10) | 5.5±0.3 | 2.9±0.1 | 112.9± 30.3 | 59.9± 18.9 | 928.8± 316.3 | 3.47±0.39 |
| Control group (n=14) | 4.8±0.5 5 ** | 2.3±0.2 ** | 5485.3± 3277.6 ** | 3010.6± 1770.6 ** | 5320.3± 9495.1 ** | 1.21±1.07 ** |
| L-Val group (n=14) | 5.1±0.4 *,# | 2.4±0.2 **,# | 3241.9± 2567.2 **,# | 1951.2± 1730.9 ** | 3040.1± 4163.3 ** | 4.32±1.74 ## |

The values are mean ± SD; * and ** represent statistical significance at levels of p < 0.05 and p < 0.01, respectively, as compared with the NT group; # and ## represent statistical significance at levels of p < 0.05 and p < 0.01, respectively, as compared with the control group; data was obtained by Student's t-test.

### Example 2: Action on Hepatic Insufficiency

Crj: Donryu male rats aged 8 - 9 weeks and weighing 250 g or so were used. During the experiment, the rats were given a dedicated feed MF (product of Oriental Yeast) and water ad libitum. After 12-h fasting, the rats were anesthetized with ether and a catheter was inserted into the right cervical vein of each animal and retained in the central vein. Subsequently, the rats were underwent 90% hepatectomy according to the method of Gaub et al. (J. Gaub et al., Hepatology, 4, 902 - 904, 1984). The catheter in the central vein was passed under the skin to connect the blade bones, fitted with a harness, passed through a protective coil and connected to a swivel (Bio-Cannula of Biomedica). The rats were transferred into a metabolic cage and subjected to the experiment under a non-anesthetized and unconstrained condition. The experimental fluides were administered constantly with the pumping speed set at 100 mL/kg/day. Immediately after the hepatectomy, 3 mL of a 20% glucose solution was administered to the rats subcutaneously; for 3 days after the hepatectomy, the rats were allowed to drink a 10% glucose solution and thereafter tap water. The experimental fluids were administered for 4 or 6 days. The treated groups were two, on being a control group administered with a lactated Ringer's injection [Lactec (registered trademark) of Otuka Seiyaku] and the other being an L-val group administered with valine (L-valine added to lactated Ringer's injection to a concentration of 16.88 g/L). A non-treated (NT) group was also set. After the end of the administration of the experimental fluids, the rats were anesthetized with ether and blood was taken from the abdominal aorta; thereafter, autopsy was done to collect liver samples.

### 2) Results

### a) Efficacy for viability of rat model of hepatic insufficiency due to 90% hepatectomy

The results are shown in Table 3.

Ninety percent hepatectomy induced a state of hepatic insufficiency in the rats and about a half of them died during observation for 6 days. However, the rat viability was significantly improved by 6-day administration of L-valine.

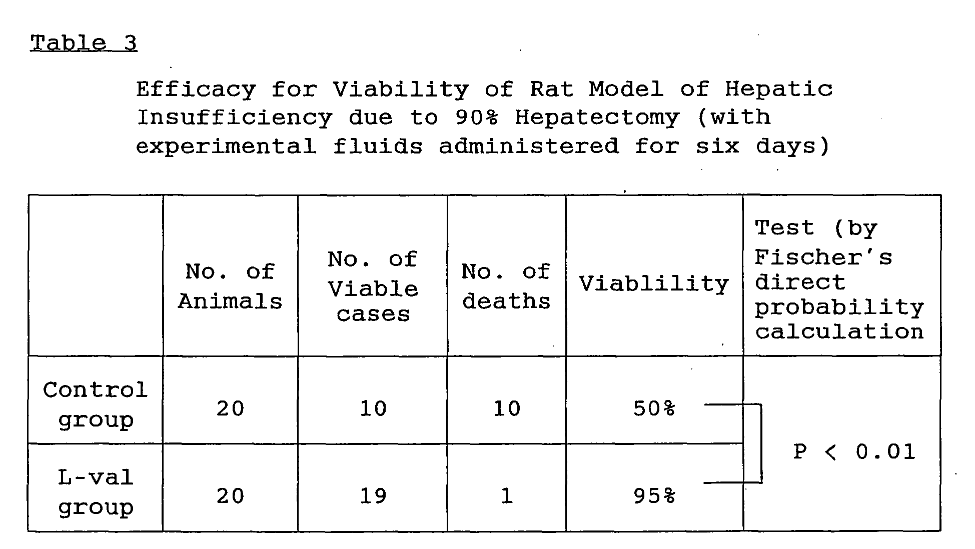

### b) Efficacy in rat model of hepatic insufficiency due to 90% hepatectomy

The results are shown in Table 4.

Ninety percent hepatectomy induced a state of hepatic insufficiency in rat but it could be improved by administration of L-valine. The Fischer ratio [BCAA (Val, Leu, Ile)/AAA (Phe, Tyr, Trp)] also improved.

**Table 4**

| Efficacy in Rat Model of Hepatic Insufficiency due to 90% Hepatectomy (with experimental fluides administered for four days) | | | | | |
|---|---|---|---|---|---|
| | TP (g/dl) | Alb (g/dl) | GOT (IU/l) | GPT (IU/l) | Fischer ratio BCAA/AAA |
| NT group (n=5) | 5.6±0.3 | 2.6±0.1 | 159.0± 35.1 | 86.2± 25.6 | 3.56±0.33 |
| Control group (n=9) | 2.8±0.8 ** | 1.5±0.3 ** | 549.9± 335.4 ** | 468.0± 82.5 ** | 0.78±0.27 ** |
| L-Val group (n=10) | 3.6±0.5 **,# | 1.8±0.2 **,# | 402.3± 302.4 ** | 134.9± 76.3 ** | 3.33±1.11 ## |

The values are mean ± SD; * and ** represent statistical significance at levels of p < 0.05 and p < 0.01, respectively, as compared with the NT group; # and ## represent statistical significance at levels of p < 0.05 and p < 0.01, respectively, as compared with the control group; data was obtained by Student's t-test.

### Example 3: Action on Chronic Hepatopathy

### 1) Experimental

Crj: Donryu male rats aged 8 - 9 weeks were used. During the experiment, the rats were given a dedicated feed MF (product of Oriental Yeast) and water ad libitum. Carbon tetrachloride (product of Wako Pure Chemical Industries) was dissolved in olive oil (product of Wako Pure Chemical Industries) to prepare a 50% (v/v) solution, which was administered subcutaneously to the rats for 14 weeks on a twice-a-week basis (to give 28 shots), each time at a dose of 2 mL/kg, thereby preparing models of chronic hepatopathy. During the 14-week experiment, the rats were given an experimental feed which was a dedicated feed MF; this was given as such to the control group and as a mixture with 3 wt% L-valine to the L-val group. A non-treated (NT) group was also set. After the end of the 14-week experiment, the rats were anesthetized with ether and blood was taken from the abdominal aorta; thereafter, autopsy was done to collect liver samples.

### 2) Results

The results are shown in Table 5.

Chronic hepatopathy was induced in the rats.

Administering carbon tetrachloride for 14 weeks induced chronic hepatopathy in the rats and some of them died. However, the hepatopathic state was ameliorated by administering the L-valine containing feed for 14 weeks.

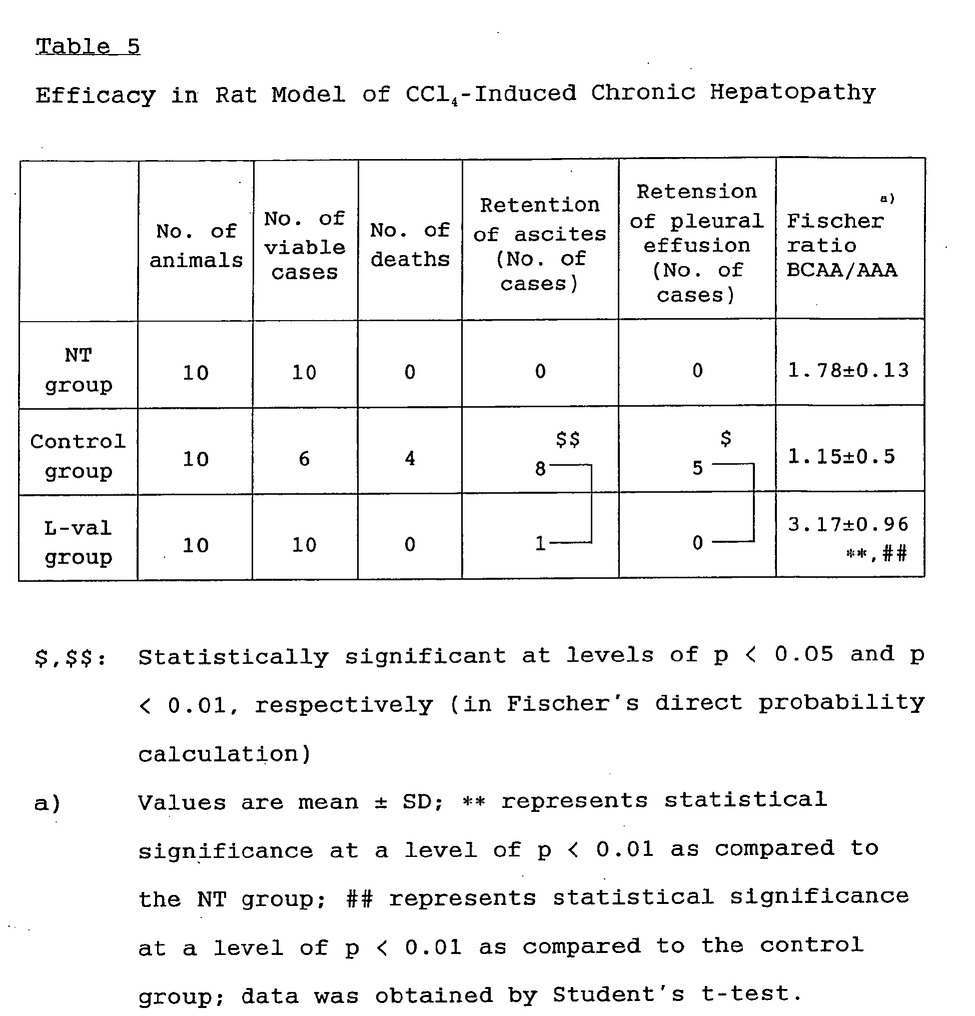

### Example 4: Efficacy of L-Valine Administered Perorally to Patients with Chronic Hepatitis C

Patient (female) with chronic hepatitis C was given oral administration of L-valine on a three-times-a-day basis, with one gram at each time. The average GOT and GPT levels of the patient for a 4-month period before the administration of L-valine were 89.0 ± 21.9 IU/L and 91.5 ± 23.1 IU/L, respectively, in terms of mean ± SD obtained by four tests on a monthly basis. The average GOT and GPT levels for a 4-month period after the start of valine administration were 68.3 ± 20.4 IU/L and 73.0 ± 24.3 IU/L, respectively, in terms of mean ± SD obtained by four tests on a monthly basis; thus, significant (p < 0.05 in t-test) decreases were observed. An ameliorating effect was also observed in platelet counts; the average platelet count for a 6-month period before the administration of L-valine was 15.7 ± 1.7 (x 10⁴) in terms of mean ± SD obtained by four tests on a monthly or bimonthly basis, whereas the average platelet count for a 4-month period after the start of valine administration was 20.4 ± 0.9 (x 10⁴) in terms of mean ± SD obtained by four tests on a monthly basis; thus, a significant (p < 0.01 in t-test) increase was observed. It was therefore clear that L-valine is also effective in patients with chronic hepatitis.

### Industrial Applicability

The composition of the present invention is a satisfactory composition for treating hepatic diseases or ameliorating the liver function in that it causes less side effects than in the conventional regimens of pharmacotherapy and that it yet can treat hepatic diseases such as acute hepatitis, hepatic insufficiency, chronic hepatitis and cirrhosis, as well as ameliorate, palliate or gain recovery from symptoms and abnormalities that are caused by such hepatic diseases, for example, fever, lassitude, loss of appetite, vomiting, stomachache, ascites and pleural effusion, or complications of hepatic disease (not including hepatic encephalopathy).

## Claims

1. A composition for treating hepatic disease or improving the liver function that contains valine as an active ingredient but which is entirely free of other amino acids or substantially free of amino other acids as an active ingredient.

2. A food composition for improving the liver function that contains valine as an active ingredient but which is entirely free of other amino acids or substantially free of amino acids as an active ingredient.

3. The composition according to 2, which is a functional food.

4. The treating or improving composition according to claim 1 or 2, wherein the hepatic disease is hepatitis.

5. The treating or improving composition according to claim 1 or 2, wherein the hepatic disease is hepatic insufficiency.

6. The treating or improving composition according to claim 1 or 2, wherein the hepatic disease is cirrhosis.

7. The treating or improving composition according to any one of claims 1 - 7, wherein valine is L-valine.

8. The treating or improving composition according to claim 1 or any one of claims 4 - 7, which is an injection.

9. The treating or improving composition according to claim 1 or any of claims 4 - 7, which is an infusion preparation.

10. The treating or improving composition according to any one of claims 1 - 7, which is an oral preparation.
